# EUROPEAN PATENT APPLICATION

(11) **EP 2 311 488 A2**
(43) Date of publication of application: **20.04.2011**
(21) Application number: 10179818.9
(22) Date of filing: 28.03.2002
(51) Int. Cl.: A61K 39/116, A61K 39/295

(54) **Vaccine composition**

(30) Priority: 03.04.2001 GB 0108364
(62) Divisional of application: 02735199.8
(71) Applicant: GlaxoSmithKline Biologicals s.a., 1330 Rixensart (BE)
(72) Inventor: Boutriau, Dominique, 1330 Rixensart (BE); Capiau, Carine, 1330 Rixensart (BE); Desmons, Pierre Michel, 1330 Rixensart (BE); Lemoine, Dominique, 1330 Rixensart (BE); Poolman, Jan, 1330 Rixensart (BE)
(74) Representative: Lubienski, Michael John

(57) **Abstract**

The present invention relates to new, advantageous DTP-based combination vaccine formulations, and concomitantly administered combination vaccine kits.

## Description

The present invention relates to new combination vaccine formulations. Combination vaccines (which provide protection against multiple pathogens) are very desirable in order to minimise the number of immunisations required to confer protection against multiple pathogens, to lower administration costs, and to increase acceptance and coverage rates. The well-documented phenomenon of antigenic competition (or interference) complicates the development of multi-component vaccines. Antigenic interference refers to the observation that administering multiple antigens often results in a diminished response to certain antigens relative to the immune response observed when such antigens are administered individually.

Combination vaccines are known which can prevent *Bordetella pertussis, Clostridium tetani, Corynebacterium diphtheriae,* and optionally Hepatitis B virus and/or *Haemophilus influenzae* type b (see, for instance, WO 93/24148 and WO 97/00697).

The present invention concerns the manufacture of the most ambitious multi-valent vaccines to date, the administration of which can prevent or treat infection by *Bordetella pertussis, Clostridium tetani, Corynebacterium diphtheriae,* Hepatitis B virus, and *N. meningitidis,* and preferably also *Haemophilus influenzae, Streptococcus pneumoniae,* Hepatitis A virus and/or Polio virus, wherein the components of the vaccine do not significantly interfere with the immunological performance of any one component of the vaccine.

Accordingly, in a one aspect of the invention there is provided a multi-valent immunogenic composition for conferring protection in a host against disease caused by *Bordetella pertussis, Clostridium tetani, Corynebacterium diphtheriae,* Hepatitis B virus, Polio virus and *N. meningitidis* comprising:
(a) either killed whole-cell *Bordetella pertussis* (Pw), or two or more acellular pertussis components (Pa) [preferably the latter],
(b) tetanus toxoid (TT or T),
(c) diphtheria toxoid (DT or D),
(d) Hepatitis B surface antigen (HepB or HB),
(e) Inactivated polio virus (IPV), and
(f) either or both conjugates of a carrier protein and a capsular polysaccharide of a bacterium selected from the group *N*. *meningitidis* type Y (MenY) and *N. meningitidis* type C (MenC), and
(g) optionally a conjugate of a carrier protein and the capsular polysaccharide *of H. influenzae* type B (Hib).

The above immunogenic composition may further comprise one, two, three, four, five, or six components selected from the following list: *N. meningitidis* type A polysaccharide [MenA] (preferably conjugated), *N. meningitidis* type W polysaccharide [MenW] (preferably conjugated), the Vi polysaccharide of *Salmonella typhi, N. meningitidis* (preferably serotype B) outer membrane vesicles, one or more *N. meningitidis* (preferably serotype B) outer membrane (surface-exposed) proteins, and killed, attenuated Hepatitis A virus (HepA - preferably the product known as 'Havrix™' [SmithKline Beecham Biologicals]) without substantial interference problems for any of the antigens of the composition.

In a second aspect of the invention there is provided various advantageous kits comprising two or three multi-valent immunogenic compositions, said kits being capable of conferring protection in a host against disease caused by *Bordetella pertussis, Clostridium tetani, Corynebacterium diphtheriae,* Hepatitis B virus, Polio virus and *Streptococcus pneumoniae,* and optionally also *N. meningitidis,* and *Haemophilus influenzae.*

In a first embodiment of the second aspect of the invention there is provided a kit comprising two multi-valent immunogenic compositions for conferring protection in a host against disease caused by *Bordetella pertussis, Clostridium tetani, Corynebacterium diphtheriae,* Hepatitis B virus, Polio virus and *Streptococcus pneumoniae,* and optionally also *N. meningitidis,* and *Haemophilus influenzae.*

The kit comprises a first container comprising:
(a) either killed whole-cell *Bordetella pertussis* (Pw), or two or more acellular pertussis components (Pa) [preferably the latter],
(b) tetanus toxoid (TT or T),
(c) diphtheria toxoid (DT or D),
(d) Hepatitis B surface antigen (HepB or HB), and
(e) Inactivated polio virus (IPV),
   and a second container comprising:
   (2a) one or more conjugates of a carrier protein and a capsular polysaccharide from Streptococcus pneumoniae [where the capsular polysaccharide is preferably from a pneumococcal serotype selected from the group consisting of 1, 2, 3, 4, 5, 6A, 6B, 7F, 8, 9N, 9V, 10A, 11A, 12F, 14, 15B, 17F, 18C, 19A, 19F, 20, 22F, 23F and 33F].

In further advantageous embodiments of the above kit of the invention, the first container additionally comprises: (f) either or both conjugates of a carrier protein and a capsular polysaccharide of a bacterium selected from the group *N. meningitidis* type Y (MenY) and *N. meningitidis* type C (MenC), and (g) a conjugate of a carrier protein and the capsular polysaccharide of *H. influenzae* type B (Hib); or the second container additionally comprises: (2b) either or both conjugates of a carrier protein and a capsular polysaccharide of a bacterium selected from the group *N. meningitidis* type Y (MenY) and *N. meningitidis* type C (MenC), and (2c) a conjugate of a carrier protein and the capsular polysaccharide of *H. influenzae* type B (Hib); or the first container additionally comprises: (f) either or both conjugates of a carrier protein and a capsular polysaccharide of a bacterium selected from the group *N. meningitidis* type Y (MenY) and *N. meningitidis* type C (MenC), and the second container additionally comprises (2b) a conjugate of a carrier protein and the capsular polysaccharide of *H*. *influenzae* type B (Hib); or the first container additionally comprises (f) a conjugate of a carrier protein and the capsular polysaccharide of *H. influenzae* type B (Hib), and the second container additionally comprises: (2b) either or both conjugates of a carrier protein and a capsular polysaccharide of a bacterium selected from the group *N*. *meningitidis* type Y (MenY) and *N. meningitidis* type C (MenC).

In a second embodiment of the second aspect of the invention there is provided a kit comprising two multi-valent immunogenic compositions for conferring protection in a host against disease caused by *Bordetella pertussis, Clostridium tetani, Corynebacterium diphtheriae,* Hepatitis B virus, Polio virus, *N. meningitidis,* and *Haemophilus influenzae.*

The kit comprises a first container comprising:
(a) either killed whole-cell *Bordetella pertussis* (Pw), or two or more acellular pertussis components (Pa) [preferably the latter],
(b) tetanus toxoid (TT or T),
(c) diphtheria toxoid (DT or D),
(d) Hepatitis B surface antigen (HepB or HB), and
(e) Inactivated polio virus (IPV),
   and a second container comprising:
   (2a) either or both conjugates of a carrier protein and a capsular polysaccharide of a bacterium selected from the group *N*. *meningitidis* type Y (MenY) and *N. meningitidis* type C (MenC), and
   (2b) a conjugate of a carrier protein and the capsular polysaccharide *of H. influenzae* type B (Hib).

In a third embodiment of the second aspect of the invention there is provided a kit comprising three multi-valent immunogenic compositions for conferring protection in a host against disease caused by *Bordetella pertussis, Clostridium tetani, Corynebacterium diphtheriae,* Hepatitis B virus, Polio virus and *N. meningitidis, Haemophilus influenzae* and *Streptococcus pneumoniae.*

The kit comprises a first container comprising:
(a) either killed whole-cell *Bordetella pertussis* (Pw), or two or more acellular pertussis components (Pa) [preferably the latter],
(b) tetanus toxoid (TT or T),
(c) diphtheria toxoid (DT or D),
(d) Hepatitis B surface antigen (HepB or HB), and
(e) Inactivated polio virus (IPV),
   and a second container comprising:
   (2a) one or more conjugates of a carrier protein and a capsular polysaccharide from Streptococcus pneumoniae [where the capsular polysaccharide is preferably from a pneumococcal serotype selected from the group consisting of 1, 2, 3, 4, 5, 6A, 6B, 7F, 8, 9N, 9V, 10A, 11A, 12F, 14, 15B, 17F, 18C, 19A, 19F, 20, 22F, 23F and 33F], and a third container comprising:
   (3a) either or both conjugates of a carrier protein and a capsular polysaccharide of a bacterium selected from the group *N*. *meningitidis* type Y (MenY) and *N. meningitidis* type C (MenC), and
   (3b) a conjugate of a carrier protein and the capsular polysaccharide *of H. influenzae* type B (Hib).

Any or the above containers of the above kits of the invention may further comprise one, two, three, four, five, six or seven components selected from the following list: *N. meningitidis* type A polysaccharide [MenA] (preferably conjugated), *N. meningitidis* type W polysaccharide [MenW] (preferably conjugated), the Vi polysaccharide of *Salmonella typhi, N. meningitidis* (preferably serotype B) outer membrane vesicles, one or more *N. meningitidis* (preferably serotype B) outer membrane (surface-exposed) proteins, HepA (as described above), and one or more S. *pneumoniae* proteins (preferably surface-exposed) without substantial interference problems for any of the antigens of the composition.

The containers of the kit can be packaged separately or, preferably, packed together. Preferably the kit is provided with a list of instructions for administration of the vaccines in the two or more containers.

Where a container in a kit contains a certain polysaccharide conjugate, it is preferred that the same conjugate is not present in the other containers of the kit.

The inventors have surprisingly found that a kit provided in the above ways advantageously presents the various antigens to a host's immune system in an optimal manner. The kit provides a medical practitioner with an optimal method of immunising a host with one or more of the following advantages (preferably 2 or 3, and most preferably all): protective efficacy for all antigens, minimal reactogenicity, minimal carrier suppression interference, minimal adjuvant/antigen interference, or minimal antigen/antigen interference. In such a way, these goals may be achieved with the minimum number (two) administrations, preferably occurring at the same visit to the practitioner.

Although in a preferred embodiment the vaccines of the first and second (and third where applicable) containers are administered concomitantly at different sites (as described later), in an alternative embodiment the inventors envision that the contents of the first and second containers may be mixed (preferably extemporaneously) before administration as a single vaccine.

### The antigens of the invention

Methods of preparing tetanus toxoid (TT) are well known in the art. For instance, TT is preferably produced by purification of the toxin from a culture of *Clostridium tetani* followed by chemical detoxification, but is alternatively made by purification of a recombinant, or genetically detoxified analogue of the toxin (for example, as described in EP 209281). 'Tetanus toxoid' also encompasses immunogenic fragments of the full-length protein (for instance Fragment C - see EP 478602).

Methods of preparing diphtheria toxoid (DT) are also well known in the art. For instance, DT is preferably produced by purification of the toxin from a culture of *Corynebacterium diphtheriae* followed by chemical detoxification, but is alternatively made by purification of a recombinant, or genetically detoxified analogue of the toxin (for example, CRM197, or other mutants as described in US 4,709,017, US 5,843,711, US 5,601,827, and US 5,917,017).

Acellular pertussis components (Pa) are well known in the art. Examples include pertussis toxoid (PT), filamentous haemagluttinin (FHA), pertactin (PRN) and agglutinogens 2 and 3. These antigens are partially or highly purified. Preferably 2 or more acellular pertussis components are used in the vaccine. More preferably 2, 3, 4 or all 5 of the above example acellular pertussis components are incorporated in the vaccine. Most preferably PT, FHA and PRN are included. PT may be produced by a variety of manners, for instance by purification of the toxin from a culture of *B. pertussis* followed by chemical detoxification, or alternatively by purification of a genetically detoxified analogue of PT (for example, as described in US 5,085,862).

Methods of preparing killed, whole-cell *Bordetella pertussis* (Pw) suitable for this invention is disclosed in WO 93/24148, as are suitable formulation methods for producing DT-TT-Pw-HepB and DT-TT-Pa-HepB vaccines.

Inactivated Polio Virus (IPV) preferably comprises types 1, 2 and 3 as is standard in the vaccine art. Most preferably it is the Salk polio vaccine.

Typically the *Streptococcus pneumoniae* vaccine of the present invention will comprise polysaccharide antigens (preferably conjugated), wherein the polysaccharides are derived from at least four serotypes of pneumococcus chosen from the group consisting of 1, 2, 3, 4, 5, 6A, 6B, 7F, 8, 9N, 9V, 10A, 11A, 12F, 14, 15B, 17F, 18C, 19A, 19F, 20, 22F, 23F and 33F. Preferably the four serotypes include 6B, 14, 19F and 23F. More preferably, at least 7 serotypes are included in the composition, for example those derived from serotypes 4, 6B, 9V, 14, 18C, 19F, and 23F. More preferably still more than 7 serotypes are included in the composition, for instance at least 11 serotypes. For example the composition in one embodiment includes 11 capsular polysaccharides derived from serotypes 1, 3, 4, 5, 6B, 7F, 9V, 14, 18C, 19F and 23F (preferably conjugated). In a preferred embodiment of the invention at least 13 polysaccharide antigens (preferably conjugated) are included, although further polysaccharide antigens, for example 23 valent (such as serotypes 1, 2, 3, 4, 5, 6B, 7F, 8, 9N, 9V, 10A, 11A, 12F, 14, 15B, 17F, 18C, 19A, 19F, 20, 22F, 23F and 33F), are also contemplated by the invention.

For elderly vaccination (for instance for the prevention of pneumonia) it is advantageous to include serotypes 8 and 12F (and most preferably 15 and 22 as well) to the preferred 11 valent antigenic composition described above to form a 13/15 valent vaccine, whereas for infants or toddlers (where otitis media is of more concern) serotypes 6A and 19A are advantageously included to form a 13 valent vaccine.

### Conjugates

The bacterial capsular polysaccharide conjugates may comprise any carrier peptide, polypeptide or protein comprising at least one T-helper epitope. Preferably the carrier protein(s) used is selected from the group comprising: tetanus toxoid, diphtheria toxoid, CRM197, recombinant diphtheria toxin (as described in any of US 4,709,017, WO 93/25210, WO 95/33481, or WO 00/48638), pneumolysin (preferably chemically detoxified, or a detoxified mutant) from *S. pneumoniae,* OMPC from *N*. *meningitidis,* and protein D from *H. influenzae* (EP 594610). Due to the known effect of carrier suppression, it is advantageous if in each of the compositions of the invention the polysaccharide antigens contained therein ('n' antigens) are conjugated to more than one carrier. Thus (n-1) of the polysaccharides could be carried (separately) on one type of carrier, and 1 on a different carrier, or (n-2) on one, and 2 on two different carriers, etc. For example, in a vaccine containing 4 bacterial polysaccharide conjugates, 1, 2 or all four could be conjugated to different carriers). Protein D, however, is advantageously used as a carrier in the compositions of the invention as it may be used for various (2, 3, 4 or more) polysaccharides in a composition without a marked carrier suppression effect. Most preferably Hib is present as a TT conjugate, pneumococcal polysaccharides are protein D, DT or CRM197 conjugates, and MenA, MenC, MenY and MenW are either TT or PD conjugates. Protein D is also a useful carrier as it provides a further antigen which can provide protection against *H. influenzae.*

The polysaccharide may be linked to the carrier protein by any known method (for example, by Likhite, U.S. Patent 4,372,945 and by Armor et al., U.S. Patent 4,474,757). Preferably, CDAP conjugation is carried out (WO 95/08348).

In CDAP, the cyanylating reagent 1-cyano-dimethylaminopyridinium tetrafluoroborate (CDAP) is preferably used for the synthesis of polysaccharide-protein conjugates. The cyanilation reaction can be performed under relatively mild conditions, which avoids hydrolysis of the alkaline sensitive polysaccharides. This synthesis allows direct coupling to a carrier protein.

### Properties of the immunogenic compositions of the invention

The immunogenic compositions of the invention are preferably formulated as a vaccine for *in vivo* administration to the host in such a way that the individual components of the composition are formulated such that the immunogenicity of individual components is not substantially impaired by other individual components of the composition. By not substantially impaired, it is meant that upon immunisation, an antibody titre (e.g. IgG) against each component is obtained which is more than 60%, preferably more than 70%, more preferably more than 80 %, still more preferably more than 90%, and most preferably more than 95-100% of the titre obtained when the antigen is administered in isolation.

Interestingly, with the kit combinations described above, it is possible, upon immunisation, to obtain antibody titres against Hib capsular polysaccharide or some pneumococcal polysaccharides approaching, or in excess of, 100% of the titre obtained when the antigen is administered in isolation.

### Vaccine formulations

The immunogenic compositions of the invention are preferably formulated as a vaccine for *in vivo* administration to the host, such that they confer an antibody titre superior to the criterion for seroprotection for each antigenic component for an acceptable percentage of human subjects. This is an important test in the assessment of a vaccine's efficacy throughout the population. Antigens with an associated antibody titre above which a host is considered to be seroconverted against the antigen are well known, and such titres are published by organisations such as WHO. Preferably more than 80% of a statistically significant sample of subjects is seroconverted, more preferably more than 90%, still more preferably more than 93% and most preferably 96-100%.

The immunogenic compositions of the invention are preferably adjuvanted. Suitable adjuvants include an aluminium salt such as aluminium hydroxide gel (alum) or aluminium phosphate, but may also be a salt of calcium, iron or zinc, or may be an insoluble suspension of acylated tyrosine, or acylated sugars, cationically or anionically derivatised polysaccharides, or polyphosphazenes.

The adjuvant may also be selected to be a preferential inducer of a TH1 type of response to aid the cell mediated branch of the immune response.

High levels of Th1-type cytokines tend to favour the induction of cell mediated immune responses to a given antigen, whilst high levels of Th2-type cytokines tend to favour the induction of humoral immune responses to the antigen.

Suitable adjuvant systems which promote a predominantly Th1 response include, Monophosphoryl lipid A or a derivative thereof, particularly 3-de-O-acylated monophosphoryl lipid A, and a combination of monophosphoryl lipid A, preferably 3-de-O-acylated monophosphoryl lipid A (3D-MPL) together with an aluminium salt. An enhanced system involves the combination of a monophosphoryl lipid A and a saponin derivative, particularly the combination of QS21 and 3D-MPL as disclosed in WO 94/00153, or a less reactogenic composition where the QS21 is quenched with cholesterol as disclosed in WO 96/33739. A particularly potent adjuvant formulation involving QS21, 3D-MPL and tocopherol in an oil in water emulsion is described in WO 95/17210. The vaccine may additionally comprise a saponin, more preferably QS21. The formulation may also comprises an oil in water emulsion and tocopherol (WO 95/17210). Unmethylated CpG containing oligonucleotides (WO 96/02555) are also preferential inducers of a TH1 response and are suitable for use in the present invention.

Aluminium salts are preferred adjuvants in the above immunogenic compositions. In particular, HepB should preferably be adsorbed onto aluminium phosphate before admixing with the other components. Pertactin is preferably adsorbed onto aluminium hydroxide before admixing with the other components. In order to minimise the levels of adjuvant (particularly aluminium salts) in the compositions of the invention, the polysaccharide conjugates may be unadjuvanted.

The present invention also provides a method for producing a vaccine formulation comprising the step of mixing the components of the vaccine together with a pharmaceutically acceptable excipient.

A particularly preferred DTPa composition of the invention (for independent use or as the contents of the first container of one of the above-described kits) comprises: TT, DT, Pa (preferably comprising PT, FHA and PRN - with PRN preferably adsorbed onto aluminium hydroxide), HepB (preferably adsorbed onto aluminium phosphate), IPV, MenC (preferably conjugated onto either protein D, TT, DT or CRM197), and, optionally, MenY (preferably conjugated onto either protein D, TT, DT or CRM197). The composition may also optionally comprise Hib (preferably conjugated onto TT and/or unadsorbed onto adjuvant). Preferably the vaccine may be supplied in 2 vials, the first containing DTPa-IPV-HepB in a liquid form, and a second containing MenC (and optionally MenY and/or Hib) in a lyophilised form, preferably in the presence of an anti-caking agent such as sucrose, lactose or trehalose. The contents of the vials may be mixed extemporaneously in a single container before administering to a host in a single administration/injection. This composition may also be used in a kit described above (the contents of the first container).

For the purpose of kits comprising a container comprising Hib (preferably conjugated onto TT and/or unadsorbed onto adjuvant) and/or either or both of MenC and MenY (preferably conjugated onto either protein D, TT, DT or CRM197 and/or unadsorbed onto adjuvant), this composition is preferably stored in a lyophilised form, preferably in the presence of an anti-caking agent such as sucrose, lactose or trehalose.

For the purpose of DTPa compositions of the invention (for independent use or as the contents of the first container of one of the above-described kits) comprising a container comprising DTPa and Hib and/or either or both of MenC and MenY, where the Hib and/or Men components are conjugated to TT, it is preferable to balance the TT content in the vaccine such that the total content of TT in a single container is not more than a critical threshold (such as 40, 45, 50, 60, 70 or 80 µg TT) to reduce, minimise or prevent TT immune interference or carrier suppression of TT conjugated polysaccharides. Preferably this threshold is 50 µg. The inventors have found that the ratio of polysaccharide:TT may be reduced in the above conjugates to 1:0.5-1.5 by weight (preferably 1: 0.6-1.2, most preferably around 1:1) to be beneficial in this respect. For instance in a DTPa-HB-IPV-Hib(TT)-MenC(TT) vaccine the amount of T in DTPa should preferably be reduced below a typical standard quantity (preferably about one to three quarters, most preferably about a half of the regular amount) to, for instance, 10-30 µg TT, preferably 20-25 µg TT. For example, if the amount of TT conjugated to Hib is around 12 µg TT, and the amount conjugated to MenC is around 5 µg TT, and the amount of unconjugated TT is 24 µg, then the total TT will be about 41 µg.

A particularly preferred Hib / pneumococcal polysaccharide composition (for independent use or as the contents of the second container of one of the above-described kits) comprises: Hib (preferably conjugated onto TT and/or unadsorbed onto adjuvant) and multiple (for instance more than 1, 2, 3, 4, 5, 6, 7, 8, 9, 10 or 11) pneumococcal polysaccharide conjugates (for instance those combinations described in the paragraph on 'the *Streptococcus pneumoniae* vaccine of the present invention' above). Most preferably 11 polysaccharides (from serotypes 1, 3, 4, 5, 6B, 7F, 9V, 14, 18C, 19F and 23F) are included. Preferably pneumococcal polysaccharides are conjugated onto PD, DT, CRM197 or TT. In a preferred embodiment, the Hib polysaccharide antigen is not adsorbed onto an adjuvant, particularly aluminium salts. Although the pneumococcal polysaccharide antigen(s) may be adjuvanted (preferably onto aluminium phosphate), they may also be not adsorbed onto an adjuvant, particularly aluminium salts. In a particular embodiment, there is no aluminium adjuvant salt present in the composition. Further antigens may be included in the compositions of the invention (for instance *N. meningitidis* Type C capsular polysaccharide conjugate [preferably conjugated onto either protein D, TT, DT or CRM197 and/or unadsorbed onto adjuvant]), however, in an alternative embodiment, Hib and pneumococcal polysaccharide conjugates are the only antigens present in the composition. In a further specific embodiment of the above formulations, the Hib and pneumococcal polysaccharides are not conjugated to the same carrier (particularly where the carrier is CRM197).

The vaccine may be supplied in one container (with the contents either in a liquid or lyophilised form), or in two vials, the first containing Hib (preferably lyophilised), the second containing the pneumococcal antigens (preferably in a liquid form). Lyophilised compositions are preferably in the presence of an anti-caking agent such as sucrose, lactose or trehalose. The contents of the vials may be mixed extemporaneously in a single container before administering to a host in a single administration/injection. With such a formulation it is possible, upon immunisation, to obtain antibody titres against Hib capsular polysaccharide approaching, or most often in excess of, 100% of the titre obtained when the antigen is administered in isolation. In preferred embodiments, no (significantly) detrimental effect occur to the pneumococcal polysaccharide conjugates (in terms of protective efficacy) in the combination as compared to their administration in isolation. This can be assessed in terms of measuring post-primary geometric mean concentrations (GMC) of anti-polysaccharide antibody 1 month after the last primary dose (primary doses being the priming administrations - usually 3 - in the first year of life). The GMC (in µg/ml) for a vaccine of the invention should be preferably over 55% (more preferably over 60, 70, 80, or 90%) of the GMC when the pneumococcal polysaccharides are administered without the Hib conjugate. Another indication that no detrimental effect has occurred is if the % of subjects with antibody concentrations of no less than 0.5 µg/ml differs by no more than 10% (preferably less than 9, 7, 5, 3 or 1%) when comparing 1 month post-primary administrations of the vaccine of the invention versus the vaccine without Hib conjugate.

Although the above refers to Hib, pneumococcal and meningococcal 'polysaccharides' it is envisaged that the invention may be extended to Hib and pneumococcal 'sized-polysaccharides' and 'oligosaccharides' (polysaccharides reduced in size for manageability, which are still capable of inducing a protective immune response in a host) which are well known in the vaccine art (see for instance EP 497525). Advantageously, MenY may be present as an oligosaccharide conjugate with the oligosaccharide 0.1, 0.2, 0.3, 0.4, 0.5, 0.6, 0.7, 0.8, or 0.9 times the molecular weight of the native polysaccharide.

In a further aspect of the present invention there is provided an immunogenic composition or vaccine as herein described for use in a medicament.

In a still further aspect of the invention there is provided a use of the immunogenic compositions of the invention in the manufacture of a medicament for the treatment or prevention of diseases caused by infection by *Bordetella pertussis, Clostridium tetani, Corynebacterium diphtheriae,* Hepatitis B virus, Polio virus and *N. meningitidis* (and optionally *H. influenzae*). Furthermore, there is provided a use of the immunogenic compositions of the invention in the manufacture of a vaccine kit for the treatment or prevention of diseases caused by infection by *Bordetella pertussis, Clostridium tetani, Corynebacterium diphtheriae,* Hepatitis B virus, Polio virus, *Haemophilus influenzae, Streptococcus pneumoniae* and *N. meningitidis.*

Additionally, a method of immunising a human host against disease caused by *Bordetella pertussis, Clostridium tetani, Corynebacterium diphtheriae,* Hepatitis B virus, Polio virus and *N. meningitidis* (and optionally *H. influenzae),* which method comprises administering to the host an immunoprotective dose of the immunogenic composition of the invention is also provided.

A further aspect of the invention concerns a method of immunising a human host against disease caused by *Bordetella pertussis, Clostridium tetani, Corynebacterium diphtheriae,* Hepatitis B virus, and Polio virus, and one or more of *Haemophilus influenzae, Streptococcus pneumoniae* and *N. meningitidis,* with the kits of the invention described above, which method involves a concomitant administration schedule as defined below.

### Concomitant administration schedule

Such a schedule comprises the step of administering to a host an immunoprotective dose of an immunogenic composition of a first container of a kit (for instance one of the kits of the invention) at a different site drained by a different lymph nodes from the site at which the immunogenic composition of the second (or third) container of the kit is administered. Preferably the different sites are different limbs. Preferably the administration of the vaccines occurs within 24 hours of each other, more preferably within the same day, and most preferably at the same visit of the host to the practitioner. Preferably, the host is subsequently primed with both (or all) vaccines in the same way one or more (preferably 2) further times, each time separated by 2-12 weeks (preferably approximately 1 month). Often a third priming administration may be given between 2 weeks and 7 months after the second administration. For instance, the vaccine may be administered as above according to a normal administration schedule for DTP vaccines (such as a three visit system, each visit separated by 1 month, for instance a 3, 4 and 5 month of age schedule; or a 3, 5 and 11; or a 3, 5 and 12 month of age schedule). Such an administration schedule allows the optimisation of the immune response against the antigens in both (or all) containers of the kit.

A booster administration of the vaccines may be given in the same way anytime from the second year of life to adulthood. Although priming is preferably done via the intramuscular route, boosting may advantageously be carried out mucosally, optionally in the presence of a mucosal adjuvant (preferably laureth 9 or Heat Labile Toxin [LT] from E. coli and mutants or fragments thereof), (for instance intranasal administration of the vaccines is easy to administer and can work extremely well especially when the host is primed parenternally), and site of administration of the vaccines need not drain to different lymph nodes.

The use of the immunogenic compositions of the invention within containers in a method of manufacturing a vaccine kit of the invention for concomitant administration is also envisaged.

### Kits comprising TT in two or more containers

A further aspect of the invention concerns vaccine kits for concomitant administration (as defined above) where the TT content of two or more containers are balanced to advantageously reduce, minimise or prevent TT immune interference or carrier suppression of TT conjugated polysaccharides. TT is an extremely good carrier, however it is known that it has limitations if used to excess in a vaccine composition, particularly if free TT is also present. If used excessively, all antigens conjugated to TT exhibit reduced antibody titres. There is therefore a distinct problem in the art of how to use TT in many different areas (for instance as free antigen and as carrier for many polysaccharide antigens) within a large combination vaccine without the above disadvantages. The present inventors have found an optimal method of solving this problem; that by using a kit concomitant administration schedule (as defined above), a vaccine in a first container comprising TT in a quantity not more than a critical threshold where immune interference or carrier suppression occurs can be administered with a vaccine in a second (and optionally third) container comprising TT in a quantity not more than a critical threshold where immune interference or carrier suppression occurs such that the total quantity of TT concomitantly administered is above this critical threshould, and immune interference (or carrier suppression) is minimised (i.e. less than if the components had been administered in one injection) and preferably does not occur at all. The critical threshold can be 40, 45, 50, 60, 70 or 80 µg TT, and is preferably about 50 µg TT. The maximum total TT that can be administered is therefore approximately up to a quantity derived from the number of containers of the kit (two or three) multiplied by the critical threshould.

The present invention therefore provides a kit comprising two (or three) containers comprising two (or three) immunogenic compositions for concomitant administration each comprising TT in a free and/or conjugated form, wherein the quantity of TT in each container is not more than a critical threshold to prevent or minimise TT immune interference (or carrier suppression) effects, but the total TT in all containers is more than said critical threshold.

Preferably at least one of the containers should include free (unconjugated) TT, most preferably in the context of a DTPa or DTPw multivalent vaccine. Although the quantity of free TT can be present at around normal levels of approximately 42 µg, a further advantage of the invention allows lower quantities to be present (10-30 or 10-20 µg, for instance 10, 15, 20, 25 or 30 µg) but optimal anti-TT antibody titres may still be elicited with minimal (or no) immune interference or carrier suppression effects.

Preferably at least one (but possibly 2 or 3) of the containers should include at least one (but possibly 2, 3, 4, 5, 6, 7 or more) TT conjugated polysaccharide. Where free TT is present in one container, it is preferred that at least one TT-conjugated polysaccharide should be in one of the other containers of the kit. The polysaccharide may be any described in this application, preferably one or more pneumococcal polysaccharides (as described above), or MenC, MenY, or Hib.

Preferably the kit is any of the kits of the invention as described above.

Preferably one, two, three or all the polysaccharide-TT conjugates present in the kit are such that the ratio of polysaccharide:TT is reduced (compared to standard conjugates) to 1:0.5-1.5 by weight (preferably 1: 0.6-1.2, most preferably around 1:1) such that the conjugates are still immunologically functional, but TT immune interference or carrier suppression effects are facilitated in being minimised or prevented.

Further provided is a method of immunising a human host using the above kit, which method comprises administering an immunoprotective dose of the immunogenic composition of the first container to the host at a first site, administering an immunoprotective dose of the immunogenic composition of the second container to the host at a second site (and optionally administering an immunoprotective dose of the immunogenic composition of the third container to the host at a third site), wherein the first and second (and third) sites are drained by different lymph nodes.

Concomitant administration should be carried out as described above. Preferably the first and second (and third) sites represent different limbs of the host. Preferably the administration of the immunogenic compositions of the first and second (and third) containers occurs on the same day. Preferably the host is subsequently vaccinated in the same way one or more further times, each time separated by 2-12 weeks, more preferably two further times, each time separated by approximately a period of 1-2 months.

### Kits comprising DT or CRM197 in two or more containers

A still further aspect of the invention concerns vaccine kits for concomitant administration (as defined above) where the DT content (including DT and any immunologically identical mutants such as CRM197) of two or more containers are balanced advantageously to enhance DT (or CRM197) conjugated polysaccharide antibody titres whilst minimising reactogenicity (i.e. lower reactogenicity than if the components of the containers were administered in a single injection). DT and CRM197 are extremely good carriers, however it is known that DT contributes largely to the reactogenicity of vaccines containing it. The present inventors have found that by using a kit concomitant administration schedule (as defined above), a vaccine in a first container comprising DT (and/or CRM197) is advantageously present in a high amount (40-150 µg, preferably 60-120 µg, more preferably 70-100 µg, most preferably around 95 µg) where a vaccine in a second (and optionally third) container comprising a DT- or CRM197-conjugated polysaccharide is concomitantly administered.

The advantages of this invention are that a) although the DT content is high in the first container it is not high enough to induce DT immune interference or carrier suppression effects, b) the DT- or CRM-197 polysaccharide conjugate is separated from the first container so that the reactogenicity of the vaccine of the first container is not increased, yet c) the antibody titre against the polysaccharide conjugated to DT or CRM197 is not reduced and may be enhanced (greater titres compared to where the conjugate is administered separately, or compared to where lower quantities of DT are present in the first container).

The present invention therefore provides a kit comprising two (or three) containers comprising two (or three) immunogenic compositions for concomitant administration (as defined above), wherein the first container comprises a DT content (DT plus CRM197; preferably free or unconjugated) which is present in a high amount (as defined above), and the second (and third) containers comprise one or more polysaccharides conjugated to DT and/or CRM197.

Preferably the first container should include free (unconjugated) DT, most preferably in the context of a DTPa or DTPw multivalent vaccine.

The DT/CRM197 conjugated polysaccharide(s) may be any described in this application; preferably one or more from the following list: pneumococcal polysaccharides 1, 2, 3, 4, 5, 6A, 6B, 7F, 8, 9N, 9V, 10A, 11A, 12F, 14, 15B, 17F, 18C, 19A, 19F, 20, 22F, 23F or 33F, MenC, MenY, or Hib. Preferably according to this invention the immune response (antibody titres) against one or more of these polysaccharides is maintained compared to administering the conjugate by itself, and is most preferably enhanced.

Preferably the kit is any of the kits of the invention as described above.

Preferably one, two, three or all the polysaccharide-DT (or CRM197) conjugates present in the kit are such that the ratio of polysaccharide:DT/CRM197 is reduced (compared to standard conjugates) to 1:0.5-1.5 by weight (preferably 1: 0.6-1.2, most preferably around 1:1).

Further provided is a method of immunising a human host using the above kit, which method comprises administering an immunoprotective dose of the immunogenic composition of the first container to the host at a first site, administering an immunoprotective dose of the immunogenic composition of the second container to the host at a second site (and optionally administering an immunoprotective dose of the immunogenic composition of the third container to the host at a third site), wherein the first and second (and third) sites are drained by different lymph nodes.

Concomitant administration should be carried out as described above. Preferably the first and second (and third) sites represent different limbs of the host. Preferably the administration of the immunogenic compositions of the first and second (and third) containers occurs on the same day. Preferably the host is subsequently vaccinated in the same way one or more further times, each time separated by 2-12 weeks, more preferably two further times, each time separated by approximately a period of 1-2 months.

The vaccine preparations of the present invention may be used to protect or treat a mammal susceptible to infection, by means of administering said vaccine via systemic or mucosal route. These administrations may include injection *via* the intramuscular, intraperitoneal, intradermal or subcutaneous routes; or *via* mucosal administration to the oral/alimentary, respiratory (e.g. intranasal), genitourinary tracts.

The amount of antigen in each vaccine dose is selected as an amount which induces an immunoprotective response without significant, adverse side effects in typical vaccines. Such amount will vary depending upon which specific immunogen is employed and how it is presented. Generally, it is expected that each dose will comprise 0.1-100 µg of polysaccharide, preferably 0.1-50 µg, preferably 0.1-10 µg, of which 1 to 5 µg is the most preferable range.

The content of protein antigens in the vaccine will typically be in the range 1-100µg, preferably 5-50µtg, most typically in the range 5 - 25µg.

Following an initial vaccination, subjects may receive one or several booster immunisations adequately spaced.

Vaccine preparation is generally described in Vaccine Design ("The subunit and adjuvant approach" (eds Powell M.F. & Newman M.J.) (1995) Plenum Press New York). Encapsulation within liposomes is described by Fullerton, US Patent 4,235,877.

Embodiments of the invention are further described in the subsequent description paragraphs:
**Paragraph 1.** A vaccine kit for concomitant administration comprising two multi-valent immunogenic compositions for conferring protection in a host against disease caused by *Bordetella pertussis, Clostridium tetani, Corynebacterium diphtheriae,* Hepatitis B virus, Polio virus and *Streptococcus pneumoniae,* said kit comprising a first container comprising:
   (a) acellular pertussis components comprising pertussis toxoid and FHA,
   (b) tetanus toxoid (TT),
   (c) diphtheria toxoid (DT),
   (d) Hepatitis B surface antigen, and
   (e) Inactivated polio virus, and a second container comprising:
      (2a) one or more conjugates of a carrier protein and a capsular polysaccharide from *Streptococcus pneumoniae.*
**Paragraph** 2. The vaccine kit of paragraph 1, wherein the one or more conjugates of a carrier protein and a capsular polysaccharide from *Streptococcus pneumoniae* is derived from one or more pneumococcal serotypes selected from the group consisting of 1, 2, 3, 4, 5, 6A, 6B, 7F, 8, 9N, 9V, 10A, 11A, 12F, 14, 15B, 17F, 18C, 19A, 19F, 20, 22F, 23F and 33F.
**Paragraph 3.** The vaccine kit of paragraph 1 or 2 wherein the first container additionally comprises: (f) either or both conjugates of a carrier protein and a capsular polysaccharide of a bacterium selected from the group *N. meningitidis* type Y (MenY) and *N. meningitidis* type C (MenC), and (g) a conjugate of a carrier protein and the capsular polysaccharide *of H. influenzae* type B (Hib).
**Paragraph 4.** The vaccine kit of paragraph 1 or 2 wherein the second container additionally comprises: (2b) either or both conjugates of a carrier protein and a capsular polysaccharide of a bacterium selected from the group *N. meningitidis* type Y (MenY) and *N. meningitidis* type C (MenC), and (2c) a conjugate of a carrier protein and the capsular polysaccharide *of H. influenzae* type B (Hib).
**Paragraph 5.** The vaccine kit of paragraph 1 or 2 wherein the first container additionally comprises: (f) either or both conjugates of a carrier protein and a capsular polysaccharide of a bacterium selected from the group *N. meningitidis* type Y (MenY) and *N. meningitidis* type C (MenC), and the second container additionally comprises (2b) a conjugate of a carrier protein and the capsular polysaccharide of *H. influenzae* type B (Hib).
**Paragraph 6.** The vaccine kit of paragraph 1 or 2 wherein the first container additionally comprises (f) a conjugate of a carrier protein and the capsular polysaccharide of *H. influenzae* type B (Hib), and the second container additionally comprises: (2b) either or both conjugates of a carrier protein and a capsular polysaccharide of a bacterium selected from the group *N. meningitidis* type Y (MenY) and *N. meningitidis* type C (MenC).
**Paragraph 7.** The vaccine kit of paragraph 1 or 2 additionally comprising a third container comprising: (3 a) either or both conjugates of a carrier protein and a capsular polysaccharide of a bacterium selected from the group *N. meningitidis* type Y (MenY) and *N. meningitidis* type C (MenC), and (3b) a conjugate of a carrier protein and the capsular polysaccharide *of H. influenzae* type B (Hib).
**Paragraph 8.** The vaccine kit of paragraphs 1-7 wherein two or more containers comprise TT, the quantity of TT in each of said two or more containers being not more than a critical threshold of 50 µg TT to prevent or minimise TT immune interference or carrier suppression effects, but the total TT in all containers of the vaccine kit being more than said critical threshold.
**Paragraph 9.** The vaccine kit of paragraph 8 wherein 1, 2 or 3 of the containers includes one or more TT-conjugated polysaccharide selected from a list consisting of a pneumococcal polysaccharide, MenC, MenY, and Hib.
**Paragraph 10.** The vaccine kit of paragraph 9 wherein one or more of the TT-conjugated polysaccharides have a ratio of polysaccharide:TT of 1:0.5-1.5 by weight.
**Paragraph 11.** The vaccine kit of paragraphs 1-10 wherein the first container comprises a DT content of 60-100 µg, and the second or third containers comprise one or more polysaccharides conjugated to DT and/or CRM197.
**Paragraph 12.** The vaccine kit of paragraph 11 wherein the one or more polysaccharides conjugated to DT and/or CRM197 are selected from a list consisting of pneumococcal polysaccharides 1, 2, 3, 4, 5, 6A, 6B, 7F, 8, 9N, 9V, 10A, 11A, 12F, 14, 15B, 17F, 18C, 19A, 19F, 20, 22F, 23F, and 33F, meningococcal polysaccharides MenC and MenY, and *H. influenzae* type b Hib.
**Paragraph 13.** The vaccine kit of paragraph 11 or 12 wherein one or more of the polysaccharides conjugated to DT and/or CRM197 have a ratio of polysaccharide:DT or CRM197 of 1:0.5-1.5 by weight.
**Paragraph 14.** A multi-valent immunogenic composition for conferring protection in a host against disease caused by *Bordetella pertussis, Clostridium tetani, Corynebacterium diphtheriae,* Hepatitis B virus, Polio virus and *N. meningitidis* comprising:
   a) acellular pertussis components comprising pertussis toxoid and FHA,
   b) tetanus toxoid,
   c) diphtheria toxoid,
   d) Hepatitis B surface antigen,
   e) Inactivated polio virus, and
   f) either or both conjugates of a carrier protein and a capsular polysaccharide of a bacterium selected from the group *N*. *meningitidis* type Y and *N. meningitidis* type C.
**Paragraph 15.** The immunogenic composition of paragraph 14 further comprising one or more conjugates of a carrier protein and a capsular polysaccharide of a bacterium selected from the group *H. influenzae* type b, *N. meningitidis* type A and *N. meningitidis* type W.
**Paragraph 16.** The immunogenic composition of paragraph 14 or 15 further comprising killed, attenuated Hepatitis A virus.
**Paragraph 17.** The immunogenic composition of paragraphs 14-16 wherein the carrier protein(s) used is selected from the group comprising: tetanus toxoid, diphtheria toxoid, CRM197, recombinant diphtheria toxin, OMPC from *N*. *meningitidis,* pneumolysin from S. *pneumoniae* and protein D from *H. influenzae.*
**Paragraph 18.** The immunogenic composition of paragraphs 14-17 formulated as a vaccine for *in vivo* administration to the host wherein the individual components of the composition are formulated such that the immunogenicity of individual components is not impaired by other individual components of the composition.
**Paragraph 19.** The immunogenic composition of paragraphs 14-17 formulated as a vaccine for *in vivo* administration to the host, which confers an antibody titre superior to the criterion for seroprotection for each antigenic component for an acceptable percentage of human subjects.
**Paragraph 20.** The immunogenic composition of paragraphs 14-19 further comprising an adjuvant.
**Paragraph 21.** The immunogenic composition of paragraph 20 wherein the adjuvant is aluminium salts.
**Paragraph 22.** The use of the immunogenic composition of paragraphs 14-21 in the manufacture of a medicament for the treatment or prevention of diseases caused by infection by *Bordetella pertussis, Clostridium tetani, Corynebacterium diphtheriae,* Hepatitis B virus, Polio virus and *N. meningitidis.*
**Paragraph 23.** A method of immunising a human host against disease caused by *Bordetella pertussis, Clostridium tetani, Corynebacterium diphtheriae,* Hepatitis B virus, Polio virus and *N. meningitidis,* which method comprises administering to the host an immunoprotective dose of the immunogenic composition of paragraphs 14-21.
**Paragraph 24.** A process for making the multi-valent immunogenic composition of paragraphs 14-21 comprising the step of mixing together the individual components.
**Paragraph 25.** A multi-valent immunogenic composition for conferring protection in a host against disease caused by *Haemophilus influenzae* and *Streptococcus pneumoniae* comprising:
   (a) a conjugate of a carrier protein and the capsular polysaccharide of *H. influenzae* type B, and
   (b) one or more conjugates of a carrier protein and a capsular polysaccharide from *Streptococcus pneumoniae.*
**Paragraph 26.** The immunogenic composition of paragraph 25, wherein said composition comprises 2 or more conjugates of a carrier protein and a capsular polysaccharide from *Streptococcus pneumoniae.*
**Paragraph 27.** The immunogenic composition of paragraph 26, wherein said composition comprises more than 7 conjugates of a carrier protein and a capsular polysaccharide from *Streptococcus pneumoniae.*
**Paragraph 28.** The immunogenic composition of any one of paragraphs 25-27 wherein the capsular polysaccharide(s) from *Streptococcus pneumoniae* is from a serotype selected from the group consisting of: 1, 2, 3, 4, 5, 6A, 6B, 7F, 8, 9N, 9V, 10A, 11A, 12F, 14, 15B, 17F, 18C, 19A, 19F, 20, 22F, 23F and 33F.
**Paragraph 29.** The immunogenic composition of paragraph 28 comprising conjugates of a carrier protein and capsular polysaccharides from *Streptococcus pneumoniae* serotypes 1, 3, 4, 5, 6B, 7F, 9V, 14, 18C, 19F and 23F.
**Paragraph 30.** The immunogenic composition of paragraphs 25-29 further comprising an adjuvant.
**Paragraph 31.** The immunogenic composition of paragraphs 25-29, wherein the capsular polysaccharide of *H. influenzae* type B is not adsorbed onto an aluminium salt adjuvant.
**Paragraph 32.** The immunogenic composition of paragraph 31, wherein the capsular polysaccharides of S. *pneumoniae* are adsorbed onto an aluminium salt adjuvant.
**Paragraph 33.** The immunogenic composition of paragraph 31, wherein both the capsular polysaccharide *of H. influenzae* type B and the capsular polysaccharides of S. *pneumoniae* are not adsorbed onto an aluminium salt adjuvant.
**Paragraph 34.** The immunogenic composition of paragraphs 25-33 wherein the carrier protein(s) used is selected from the group comprising: tetanus toxoid, diphtheria toxoid, CRM197, recombinant diphtheria toxin, OMPC from *N*. *meningitidis,* pneumolysin from S. *pneumoniae* and protein D from *H. influenzae.*
**Paragraph 35.** The immunogenic composition of paragraphs 25-34, wherein the capsular polysaccharide of *H. influenzae* type B and the capsular polysaccharide from *Streptococcus pneumoniae* are not conjugated to the same carrier.
**Paragraph 36.** The immunogenic composition of paragraph 35, wherein the capsular polysaccharide of *H. influenzae* type B and the capsular polysaccharide from *Streptococcus pneumoniae* are not all conjugated to CRM197.
**Paragraph 37.** The immunogenic composition of paragraph 34 or 35, wherein the carrier protein for the capsular polysaccharide of *H. influenzae* type B is tetanus toxoid.
**Paragraph 38.** The immunogenic composition of paragraph 34, 35 or 37 wherein the carrier protein for all the capsular polysaccharide of S. *pneumoniae* is protein D.
**Paragraph 39.** The immunogenic composition of paragraphs 25-38 formulated as a vaccine for *in vivo* administration to the host wherein the individual components of the composition are formulated such that the immunogenicity of individual components is not impaired by other individual components of the composition.
**Paragraph 40.** The immunogenic composition of paragraphs 25-38 formulated as a vaccine for *in vivo* administration to the host, which confers an antibody titre superior to the criterion for seroprotection for each antigenic component for an acceptable percentage of human subjects.
**Paragraph 41.** The immunogenic composition of paragraphs 25-40 for use in a medicament.
**Paragraph 42.** The use of the immunogenic composition of paragraphs 25-40 in the manufacture of a medicament for the treatment or prevention of diseases caused by infection by *Haemophilus influenzae* and *Streptococcus pneumoniae.*
**Paragraph 43.** A method of immunising a human host against disease caused by *Haemophilus influenzae* and *Streptococcus pneumoniae,* which method comprises administering to the host an immunoprotective dose of the immunogenic composition of paragraphs 25-40.
**Paragraph 44.** A process for making the multi-valent immunogenic composition of paragraphs 25-40 comprising the step of mixing together the individual components.
**Paragraph 45.** A kit comprising two multi-valent immunogenic compositions for conferring protection in a host against disease caused by *Bordetella pertussis, Clostridium tetani, Corynebacterium diphtheriae,* Hepatitis B virus, Polio virus and *N. meningitidis, Haemophilus influenzae* and *Streptococcus pneumoniae,* wherein said kit comprises a first container comprising the immunogenic composition of paragraphs 14-21, and a second container comprising the immunogenic composition of paragraphs 25-40.
**Paragraph 46.** A method of immunising a human host against disease using the kit of paragraphs 1-13 or 45, which method comprises administering an immunoprotective dose of the immunogenic composition of the first container to the host at a first site, administering an immunoprotective dose of the immunogenic composition of the second container to the host at a second site, and, where relevant, administering an immunoprotective dose of the immunogenic composition of the third container to the host at a third site, wherein the first, second and third sites are drained by different lymph nodes.
**Paragraph 47.** The method of paragraph 46, wherein the first, second and, where relevant, third sites represent different limbs of the host.
**Paragraph 48.** The method of paragraph 46 or 47 wherein the administration of the immunogenic compositions of the first, second and, where relevant, third containers occurs on the same day.
**Paragraph 49.** The method of paragraph 46-48, wherein the host is subsequently vaccinated in the same way one or more further times, each time separated by 2-12 weeks.
**Paragraph 50.** The method of paragraph 49, wherein the host is subsequently vaccinated in the same way two further times, each time separated by approximately a period of 1-2 months.

### EXAMPLES

Examples are provided solely for the purposes of illustration and are not intended to limit the scope of the invention.

### Example 1: Preparation of a DT-TT-Pa-IPV-HepB (DTPaIPVHepB) Vaccine

This was done as described in WO 93/24148. The vaccine is commercially available under the name Infanrix-PeNTa™ (SmithKline Beecham Biologicals).

### Example 2: Preparation of a MenC or MenC-MenY Vaccine

MenC: *N. meningitidis* type C capsular polysaccharide conjugated onto either protein D or TT (using the CDAP technique) present in an amount of 5 µg of polysaccharide in the conjugate per 0.5 mL human dose. The pH was adjusted to 6.1, and was lyophilised in the presence of sucrose.

MenCMenY: *N. meningitidis* type C capsular polysaccharide conjugated onto either protein D or TT (using the CDAP technique) and *N. meningitidis* type Y capsular polysaccharide conjugated onto either protein D or TT were mixed together in an amount of 5 µg of polysaccharide in each conjugate per 0.5 mL human dose. The pH was adjusted to 6.1, and was lyophilised in the presence of sucrose.

### Example 3: Preparation of a DT-TT-Pa-IPV-HepB-MenC-MenY (DTPaIPVHepB/MenCMenY) or a DT-TT-Pa-IPV-HepB-MenC (DTPaIPVHepB/MenC) Vaccine

The vaccines of Example 1 and Example 2 were mixed extemporaneously (on the same day) before use.

### Example 4: Preparation of a Hib - 11 valent pneumococcal conjugate (Hib/Strep11V) Vaccine

*H. influenzae* type b capsular polysaccharide conjugated onto TT (10 µg of polysaccharide in the conjugate per dose) which had been lyophilised at a pH of 6.1 in the presence of lactose [Hiberix™ (SmithKline Beecham Biologicals)] was extemporaneously (on the same day as use) dissolved in a liquid solution of eleven-valent pneumococcal capsular polysaccharide (serotypes 1, 3, 4, 5, 6B, 7F, 9V, 14, 18C, 19F and 23F) conjugated onto PD (1 µg of polysaccharide in each conjugate per dose). The pneumococcal vaccine had previously been adsorbed onto 0.5 mg Al³⁺ (as AlPO₄).

### Example 5: Clinical Trials

### Studies on the Vaccine of Example 4

The vaccine of Example 4 and a control vaccine were administered in a three-dose (3, 4, 5 months of age) schedule to German infants.

The immune response results (measured 1 month after the last primary administration) were as follows.

Anti pneumococcal IgG antibodies: GMC (µg/ml) (ByElisa)

| PS | | Group A | | | Group D | | |
|---|---|---|---|---|---|---|---|
| Anti-body | Timing | N | S+[%] | GMC | N | S+[%] | GMC |
| Anti-1 | PIII | 30 | 100 | 1.23 | 33 | 100 | 0.99 |
| Anti-3 | PIII | 30 | 100 | 2.04 | 33 | 97.0 | 1.20 |
| Anti-4 | PIII | 30 | 100 | 0.98 | 33 | 100 | 1.03 |
| Anti-5 | PIII | 30 | 100 | 1.33 | 33 | 100 | 1.34 |
| Anti-6B | PIII | 30 | 100 | 0.54 | 33 | 100 | 0.62 |
| Anti-7F | PIII | 30 | 100 | 1.60 | 33 | 100 | 1.33 |
| Anti-9V | PIII | 30 | 100 | 1.61 | 33 | 100 | 1.21 |
| Anti-14 | PIII | 30 | 100 | 2.27 | 33 | 100 | 2.32 |
| Anti-18C | PIII | 30 | 100 | 1.06 | 33 | 100 | 1.04 |
| Anti-19F | PIII | 30 | 100 | 2.05 | 33 | 100 | 1.92 |
| Anti-23F | PIII | 30 | 96.7 | 0.75 | 33 | 100 | 0.76 |

| | | | | | | | |
|---|---|---|---|---|---|---|---|
| Group A= 11Pn-PD + Irifanrix-HeXa™ (Infanrix-Penta plus added Hib conjugate - DTPa-HB-IPV-Hib) Group D = 11Pn-PD/Hib + Infanrix-PeNTa™ (DTPa-HB-IPV) + indicates concomitant (in different limbs) rather than combined administration. | | | | | | | |

Percent of subjects with antibody concentrations no less than 0.5 µg/ml

| group | PS 1 | 3 | 4 | 5 | 6B | 7F | 7V | 14 | 18C | 19F | 23F |
|---|---|---|---|---|---|---|---|---|---|---|---|
| D | 84.8 | 87.9 | 87.9 | 90.9 | 51.5 | 90.9 | 93.9 | 97.0 | 81.8 | 97.0 | 72.7 |
| A | 86.7 | 96.7 | 76.7 | 90.0 | 50.0 | 93.3 | 90.0 | 90.0 | 80.0 | 96.7 | 66.7 |

Anti PRP antibodies: GMC (µg/ml) (By Elisa)

| | | Group D (N = 34) | | |
|---|---|---|---|---|
| | | n | ≥1 µg/ml [%] | GMC [µg/ml] |
| Anti-PRP | PIII | 33 | 100 | 10.75 |

| | | | | |
|---|---|---|---|---|
| 100% of subjects had anti-PRP (Hib polysaccharide) antibody concentrations no less than 1.0 µg/ml. | | | | |

Hiberix (unadsorbed Hib-TT conjugate) has a GMC after a similar administration schedule of about 6 µg/ml.

The immune response, in terms of ELISA antibodies, of infants who received the 11Pn-PD/Hib vaccine was similar to that observed for those who received the 11Pn-PD vaccine for all of the serotypes, with the exception of serotypes 1, 3 and 9V for which a trend to lower geometric mean concentrations was observed for the 11Pn-PD/Hib vaccine. However, these differences were not significant as shown by the overlapping of 95% confidence intervals.

The 11Pn-PD/Hib vaccine induced functional (opsonophagocytic) antibodies to all 11 serotypes.

Combining the Hib vaccine with the pneumococcal conjugate vaccine did not significantly interfere with the pneumococcal immune response and surprisingly enhanced the anti PRP response compared to both the registered vaccines Infanrix-HeXa and Hiberix.

### Studies on the Vaccines of Example 3, or the Concomitant Administration of the Vaccines of Example 3 and Example 4

### Study 1:

The safety and immunogenicity of Infanrix-PeNTa mixed with MenC conjugate vaccine given with a Hib vaccine or concomitantly with 11-valent pneumococcal vaccine mixed with Hiberix can be evaluated. Both PD and TT carriers can be evaluated for the MenC conjugate. The vaccines can be administered as a three dose vaccine in infants. Concomitant injection can be in different limbs, administered in the same visit to the practitioner.

### Study 2:

The safety and immunogenicity of Infanrix-PeNTa mixed with MenC-MenY conjugate vaccine given with a Hib vaccine or concomitantly with 11-valent pneumococcal vaccine mixed with Hiberix can be evaluated. Both PD and TT carrier can be evaluated for the MenC and MenY conjugates. The vaccines can be administered as a three dose vaccine in infants. Concomitant injection can be in different limbs, administered in the same visit to the practitioner.

## Claims

1. A multi-valent immunogenic composition for conferring protection in a host against disease caused by *Bordetella pertussis, Clostridium tetani, Corynebacterium diphtheriae,* Hepatitis B virus, Polio virus and *N. meningitidis* comprising:
a) acellular pertussis components comprising pertussis toxoid and FHA,
b) tetanus toxoid,
c) diphtheria toxoid,
d) Hepatitis B surface antigen,
e) Inactivated polio virus, and
f) either or both conjugates of a carrier protein and a capsular polysaccharide or oligosaccharide of a bacterium selected from the group *N. meningitidis* type Y and *N. meningitidis* type C.

2. The immunogenic composition of claim 1 further comprising one or more conjugates of a carrier protein and a capsular polysaccharide or oligosaccharide of a bacterium selected from the group *H. influenzae* type b, *N. meningitidis* type A and *N. meningitidis* type W.

3. The immunogenic composition of claim 1 or 2 further comprising killed, attenuated Hepatitis A virus.

4. The immunogenic composition of claims 1-3 wherein the carrier protein(s) used is selected from the group comprising: tetanus toxoid, diphtheria toxoid, CRM197, recombinant diphtheria toxin, OMPC from *N. meningitidis,* pneumolysin from S. *pneumoniae* and protein D from *H. influenzae.*

5. The immunogenic composition of claims 1-4 formulated as a vaccine for *in vivo* administration to the host wherein the individual components of the composition are formulated such that the immunogenicity of individual components is not impaired by other individual components of the composition.

6. The immunogenic composition of claims 1-4 formulated as a vaccine for *in vivo* administration to the host, which confers an antibody titre superior to the criterion for seroprotection for each antigenic component for an acceptable percentage of human subjects.

7. The immunogenic composition of claims 1-6 further comprising an adjuvant.

8. The immunogenic composition of claims 1-7 for use in the treatment or prevention of diseases caused by infection by *Bordetella pertussis, Clostridium tetani, Corynebacterium diphtheriae,* Hepatitis B virus, Polio virus and *N. meningitidis.*

9. A process for making the multi-valent immunogenic composition of claims 1-7 comprising the step of mixing together the individual components.

10. A vaccine kit for concomitant administration comprising two multi-valent immunogenic compositions for conferring protection in a host against disease caused by *Bordetella pertussis, Clostridium tetani, Corynebacterium diphtheriae,* Hepatitis B virus, Polio virus and *Streptococcus pneumoniae,* said kit comprising a first container comprising:
(a) acellular pertussis components comprising pertussis toxoid and FHA,
(b) tetanus toxoid (TT),
(c) diphtheria toxoid (DT),
(d) Hepatitis B surface antigen, and
(e) Inactivated polio virus,
and a second container comprising:
(2a) one or more conjugates of a carrier protein and a capsular polysaccharide or oligosaccharide from *Streptococcus pneumoniae.*

11. A kit comprising two multi-valent immunogenic compositions for conferring protection in a host against disease caused by *Bordetella pertussis, Clostridium tetani, Corynebacterium diphtheriae,* Hepatitis B virus, Polio virus, *N. meningitidis,* and *Haemophilus influenza,* said kit comprising a first container comprising:
(a) either killed whole-cell *Bordetella pertussis* (Pw), or two or more acellular pertussis components (Pa),
(b) tetanus toxoid (TT or T),
(c) diphtheria toxoid (DT or D),
(d) Hepatitis B surface antigen (HepB or HB), and
(e) Inactivated polio virus (IPV),
and a second container comprising:
(2a) either or both conjugates of a carrier protein and a capsular polysaccharide or oligosaccharide of a bacterium selected from the group *N. meningitidis* type Y (MenY) and *N. meningitidis* type C (MenC), and
(2b) a conjugate of a carrier protein and the capsular polysaccharide or oligosaccharide ofH. *influenzae* type B (Hib).

12. A multi-valent immunogenic composition for conferring protection in a host against disease caused by *Haemophilus influenzae* and *Streptococcus pneumoniae* comprising:
(a) a conjugate of a carrier protein and the capsular polysaccharide or oligosaccharide *of H. influenzae* type B, and
(b) one or more conjugates of a carrier protein and a capsular polysaccharide or oligosaccharide from *Streptococcus pneumoniae.*

13. A kit comprising two multi-valent immunogenic compositions for conferring protection in a host against disease caused by *Bordetella pertussis, Clostridium tetani, Corynebacterium diphtheriae,* Hepatitis B virus, Polio virus and *N. meningitidis, Haemophilus influenzae* and *Streptococcus pneumoniae,* wherein said kit comprises a first container comprising the immunogenic composition of claims 1-7, and a second container comprising the immunogenic composition of claim 12.

14. A kit comprising two (or three) containers comprising two (or three) immunogenic compositions for concomitant administration each comprising TT in a free and/or conjugated form, wherein the quantity of TT in each container is not more than a critical threshold of 40, 45, 50, 60, 70 or 80 µg TT to prevent or minimise TT immune interference (or carrier suppression) effects, but the total TT in all containers is more than said critical threshold.

15. A kit comprising two (or three) containers comprising two (or three) immunogenic compositions for concomitant administration, wherein the first container comprises a DT content (DT plus CRM197) of 40-150 µg, and the second (and third) containers comprise one or more polysaccharides conjugated to DT and/or CRM197.
